# EUROPEAN PATENT APPLICATION

(11) **EP 2 466 295 A2**
(43) Date of publication of application: **20.06.2012**
(21) Application number: 11191601.1
(22) Date of filing: 01.12.2011
(51) Int. Cl.: G01N 23/02

(54) **Method and apparatus for laminography inspection**

(30) Priority: 15.12.2010 US 969350
(71) Applicant: General Electric Company, Schenectady, NY 12345 (US)
(72) Inventor: Mishra, Debasish, Niskayuna, NY New York 12309 (US); Ross, William Robert, Niskayuna, NY New York 12309 (US); Hopkins, Forrest Frank, Niskayuna, NY New York 12309 (US); Frutschy, Kristopher John, Niskayuna, NY New York 12309 (US); Bueno, Clifford, Niskayuna, NY New York 12309 (US)
(74) Representative: Bedford, Grant Richard

(57) **Abstract**

An imaging system (10), in some embodiments, includes a multiple focal spot x-ray source (14) configured to generate at least two x-ray beams (26) and to project each of the generated x-ray beams (26) at a different angle one at a time toward a field of view without substantial rotation or translation of the multiple focal spot x-ray source (14). The system (10) may also include a detector (20) opposite the multiple focal spot x-ray source (14) relative to the field of view and configured to receive at least a fraction of the projected x-ray beams (26) from each of the different angles and to produce at least two x-ray projection images of the field of view corresponding to each of the different angles, wherein each of the x-ray projection images are configured to be shifted with respect to one another and added to reconstruct a plane of the field of view.

## Description

The present disclosure relates generally to laminography inspection systems and, more particularly, to laminography inspection systems with stationary multiple focal spot x-ray sources.

Many industrial applications rely on radiological inspection techniques to determine the quality of industrial parts, such as pipes, pipe arrays, fan blades, wind blade spar caps, and so forth. Such inspection techniques may also be utilized to determine one or more features of an object, such as to determine the wall thickness of a pipe. Since these industrial applications often require inspection of an entire object for quality control purposes, the x-ray sources typically employed in such applications are associated with a mechanical gantry. Each time the mechanical gantry moves the x-ray source to a new location, another image is taken, and a series of such images is typically used to determine the presence or absence of a defect in the part.

Unfortunately, the gantries associated with these single spot x-ray sources are often complex and slow, thus reducing efficiency by increasing the amount of time required to accept or reject a manufactured object. Additionally, the complexity of such systems may lead to downtime associated with necessary repairs and malfunctions. Furthermore, such complex systems may be associated with a high monetary cost and a limited field of view. Accordingly, there exists a need for improved laminography inspection systems and methods that overcome such drawbacks.

In one embodiment of the present invention, there is provided an imaging system includes a multiple focal spot x-ray source adapted to generate at least two x-ray beams and to project each of the generated x-ray beams at a different angle, one at a time, toward an object without substantial rotation or translation of the multiple focal spot x-ray source. The multiple focal spot x-ray source is disposed on a first side of the object. The imaging system also includes a detector disposed on a second side of the object opposite the first side. The detector is adapted to receive at least a fraction of the projected x-ray beams from each of the different angles after being attenuated by the object and to produce at least two x-ray projection images of the object corresponding to each of the different angles. Each of the x-ray projection images are adapted to be shifted with respect to one another and added to reconstruct a plane of the object.

In another embodiment, a laminography inspection method includes providing an object to an inspection area, irradiating the object with at least two x-ray beams from at least two different angles, one at a time, to generate a series of angularly displaced images of the object. The x-ray beams are generated by a stationary multiple focal spot x-ray source. The method also includes shifting each of the angularly displaced images with respect to one another, adding each of the shifted images together to reconstruct an image of a plane of the object, and inspecting the reconstructed image plane to identify the presence or absence of a defect in the object.

In another embodiment, a laminography inspection system includes a multiple focal spot x-ray source adapted to irradiate an object with a series of angularly displaced x-ray beams, one at a time, without substantial rotation or translation of the multiple focal spot x-ray source. The multiple focal spot x-ray source is disposed on a first side of the object. The laminography inspection system also includes a detector adapted to receive at least a fraction of the angularly displaced x-ray beams after being attenuated by the object to produce at least two x-ray projection images of the object. The detector is disposed on a second side of the object opposite the first side. The laminography inspection system also includes a processor adapted to shift and add the at least two x-ray projection images to bring at least two planes of the object into focus, one at a time.

Various features, aspects, and advantages of the present invention will become better understood when the following detailed description is read with reference to the accompanying drawings in which like characters represent like parts throughout the drawings, wherein:
FIG. 1 illustrates an embodiment of a laminography imaging system including a substantially stationary multiple focal spot x-ray source;
FIG. 2 illustrates a front view of the imaging system of FIG. 1 with a pipe positioned for imaging in accordance with embodiments of the present invention;
FIG. 3 illustrates the imaging system of FIG. 2 after generation of a first x-ray beam in accordance with embodiments of the present invention;
FIG. 4 illustrates the imaging system of FIG. 2 after generation of a second x-ray beam in accordance with embodiments of the present invention;
FIG. 5 illustrates the imaging system of FIG. 2 after generation of a third x-ray beam in accordance with embodiments of the present invention;
FIG. 6 illustrates a front view of the imaging system of FIG. 1 with a pipe array positioned for imaging in accordance with embodiments of the present invention;
FIG. 7 illustrates the imaging system of FIG. 6 after generation of a first x-ray beam in accordance with embodiments of the present invention;
FIG. 8 illustrates the imaging system of FIG. 6 after generation of a second x-ray beam in accordance with embodiments of the present invention;
FIG. 9 illustrates the imaging system of FIG. 6 after generation of a third x-ray beam in accordance with embodiments of the present invention;
FIG. 10 illustrates an embodiment of the multiple focal spot x-ray source shown in FIGS. 1-9;
FIG. 11 illustrates a laminography inspection method that may be utilized to operate the imaging system of FIG. 1 in accordance with embodiments of the present invention; and
FIG. 12 illustrates a processing method that maybe utilized to process the image data acquired during the method of FIG. 11 in accordance with embodiments of the present invention.

As described in detail below, embodiments of imaging systems including a substantially stationary multiple focal spot x-ray source that generates angularly displaced x-rays that irradiate an object before being detected by a detector are provided. Such systems may be capable of remaining substantially stationary while obtaining a complete set of planar images of an object over an arc; the set of planar images may be utilized to reconstruct slices at different planes in the object. For example, in one embodiment, a processor of a laminography inspection system may utilize the set of planar images to reconstruct slices of a pipe, determine a wall thickness of a pipe, and determine the presence and location of a defect in the pipe. Further, in some embodiments, the slices of the object (e.g., a pipe) may be reconstructed via a shift-and-add procedure and, subsequently, mathematical deblurring techniques may be used to improve the image quality and slice sensitivity profile.

The foregoing features of embodiments of the present invention may offer advantages over existing single focal spot x-ray source inspection systems. For example, the use of a multiple focal spot x-ray source may increase the speed of the inspection process. That is, multiple focal spot x-ray sources are capable of rapidly obtaining multiple images by activating each focal spot, one at a time, in rapid succession, thereby eliminating the need for gantry movement time. Each of the focal spots in such an x-ray source is electronically addressable in a specific manner, thus allowing each focal spot to be activated and deactivated quickly (e.g., within 1 microsecond, within 100 microseconds, within 1000 microseconds, etc.). Such features may reduce the length of time necessary to detect and characterize a defect in an industrial product.

Furthermore, the systems disclosed herein may offer additional advantages over single spot x-ray sources, such as the scalability of the generated power. For instance, in one embodiment, each focal spot of the multiple focal spot x-ray source may have an average power of approximately 600 Watts and, accordingly, a ten focal spot x-ray source may have a total power of approximately 6000 Watts. Such a total power may be approximately six times greater than many typical single spot x-ray sources. Indeed, such multiple focal spot x-ray sources may offer many distinct advantages over traditional single spot systems.

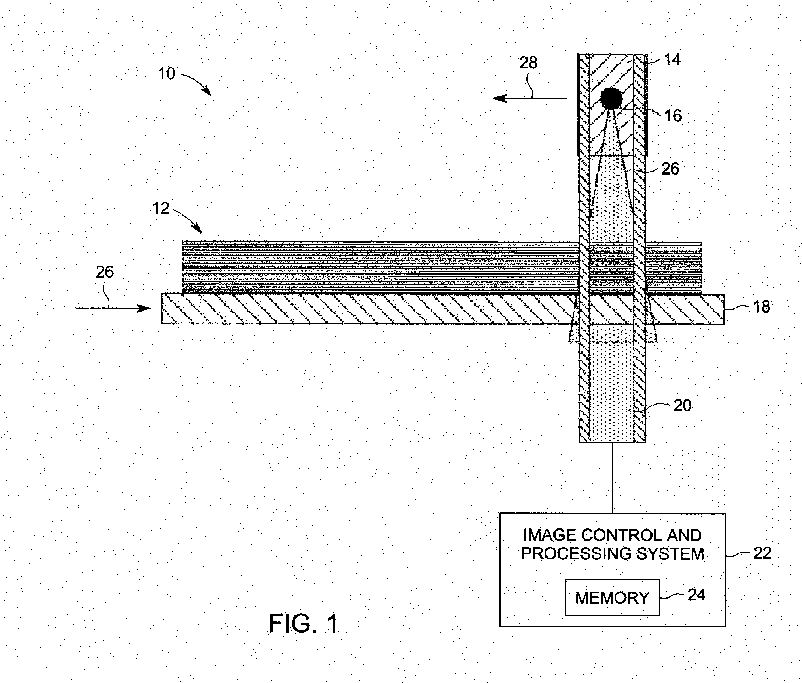

Turning now to the drawings, FIG. 1 illustrates a laminography imaging system 10 that is adapted to obtain a variety of projection images of an object 12 over an angular range without substantial motion of a multiple focal spot x-ray source 14. To that end, the system includes the multiple focal spot x-ray source 14 with focal spot 16, the object 12 disposed on an inspection board 18 in a field of view of the x-ray source, a detector 20, and an image control and processing system 22 including memory 24. In the illustrated embodiment, an x-ray beam 26 is shown projected from the focal spot 16, into the field of view, and through the object 12 onto the detector 20. However, it should be noted that although in FIG. 1, only a single focal spot 16 is shown, multiple focal spots may be located adjacent to focal spot 16 in some embodiments, as described in more detail below. In such embodiments, each focal spot may be configured to function as a module providing a distinct field of view, and, accordingly, a multiple focal spot source may be used in a plane to increase the overall field of view as compared to single focal spot sources.

During operation, the multiple focal spot source 14 generates the x-ray beam 26 from the first focal spot 16. The x-ray beam 26 is projected onto the object 12, which attenuates the beam 26 before it reaches the detector 20. As such, the detector 20 receives a fraction of the projected x-ray beam 26 after being attenuated by the object 12. In the illustrated embodiment, the detector 20 is a flat panel digital detector that digitizes the received converted x-ray energy and exports such digitized data to the image control and processing system 22. The image control and processing system 22 is adapted to convert the digitized data into a first projection image and to store the first projection image to the memory 24 for future retrieval and/or processing. In some embodiments, the image control and processing system 22 may process the first projection image before storing it to memory.

Subsequently, while remaining substantially stationary, the multiple focal spot x-ray source 14 generates additional x-ray beams, one at a time, from distinct focal spots disposed over a predefined arc. That is, without substantial rotational or translational movement, the multiple focal spot x-ray source projects x-ray beams at a variety of different angular positions. As before, each of the x-ray beams originate from a focal spot in the substantially stationary x-ray source 14, project through the object 12, and impinge the detector 20, where they are each converted to a separate projection image acquired at a different angle about the predefined arc. The generated set of planar images obtained over the arc may then be utilized by the image control and processing system 22 to reconstruct slices of the object 12 at different planes.

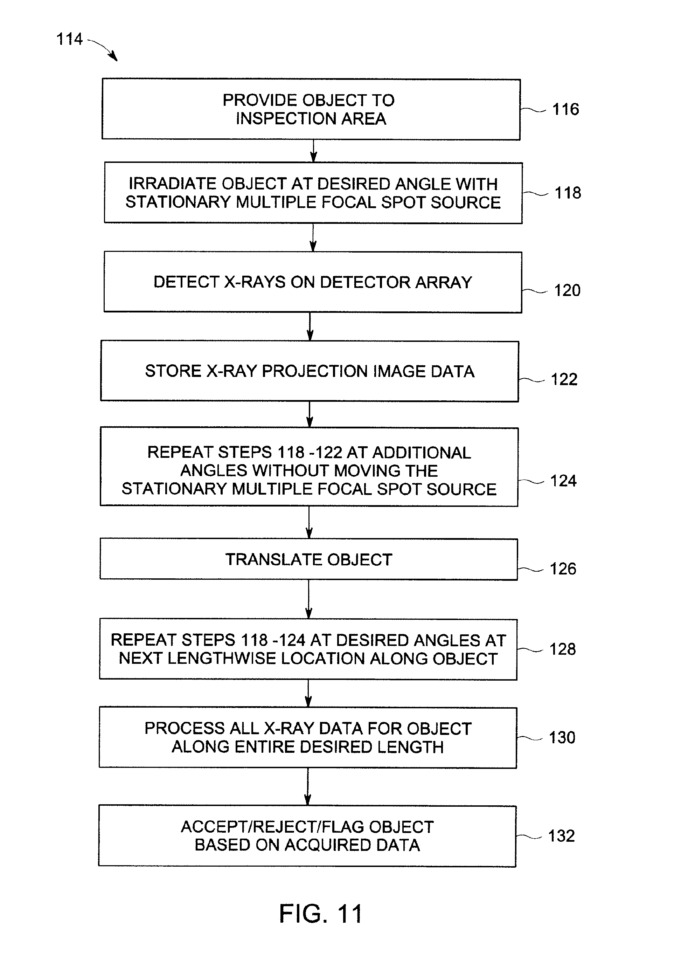

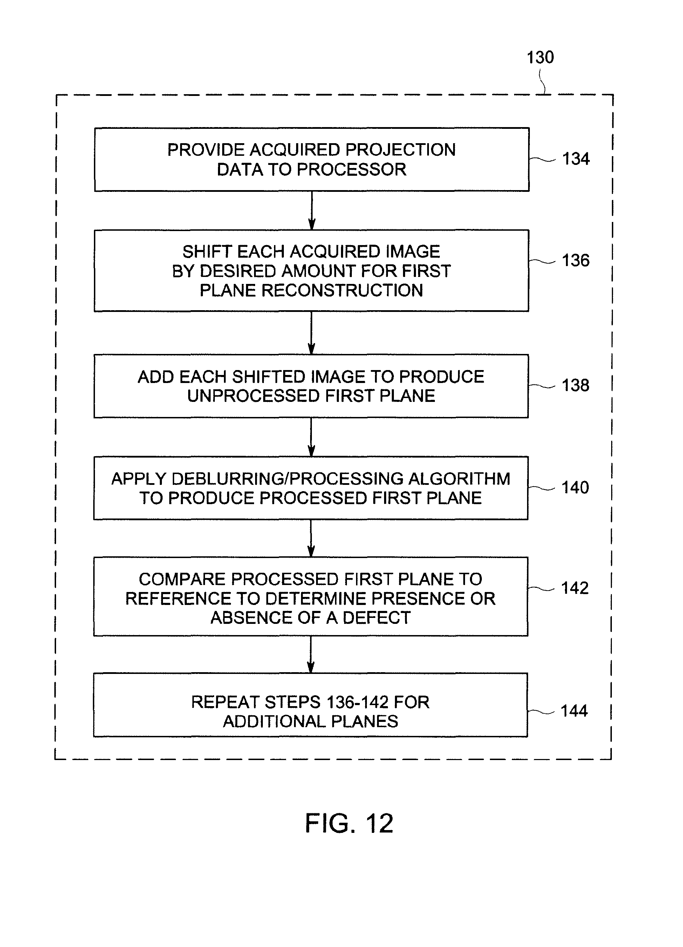

For example, in one embodiment, the digital projection images acquired at distinct angular positions are shifted and then summed a desired number of times to bring multiple depths of the object into focus, one at a time. In this embodiment, different shift distances may be utilized to reconstruct different planes in the volume of the object 12. For example, by adding the acquired projection images without applying a shift distance, the focal plane of the object 12 that is coincident with the focal plane of the scan may be reconstructed by the image processing system 22. For further example, by shifting each image by another set of first distances, which are determined based on the system geometry, and subsequently adding the shifted images, a second focal plane of the object 12 may be brought into focus. Using this approach, the image processing system 22 may reconstruct all the planes of interest in the acquisition volume. Subsequently, the processing system 22 may apply one or more mathematical deblurring techniques to each of the reconstructed planes to improve the image quality by removing image artifacts from out of focus planes. As described in more detail below with respect to FIGS. 11 and 12, such reconstructed and processed image slices may be further utilized by the control and processing system 22, for example, to determine the presence or absence of one of more defects in the object 12.

In some embodiments, after a set of projection images are acquired over the predefined arc, the object 12 may be translated, as indicated by arrow 26, and the described procedure may be repeated at the next location along the length of the object. In further embodiments, the multiple focal spot x-ray source 14 may be translated, as indicated by arrow 28, to another location along the length of the object, and the described procedure may be repeated. That is, a second set of images may be acquired over the given arc at the next lengthwise position of the object 12 and the additional image set may be used to reconstruct additional slices at distinct planes in the object. However, each time the multiple focal spot x-ray source 14 is positioned to image the object, the source 14 remains stationary (i.e., does not rotate or translate) while acquiring a complete set of projection images over the desired angular range.

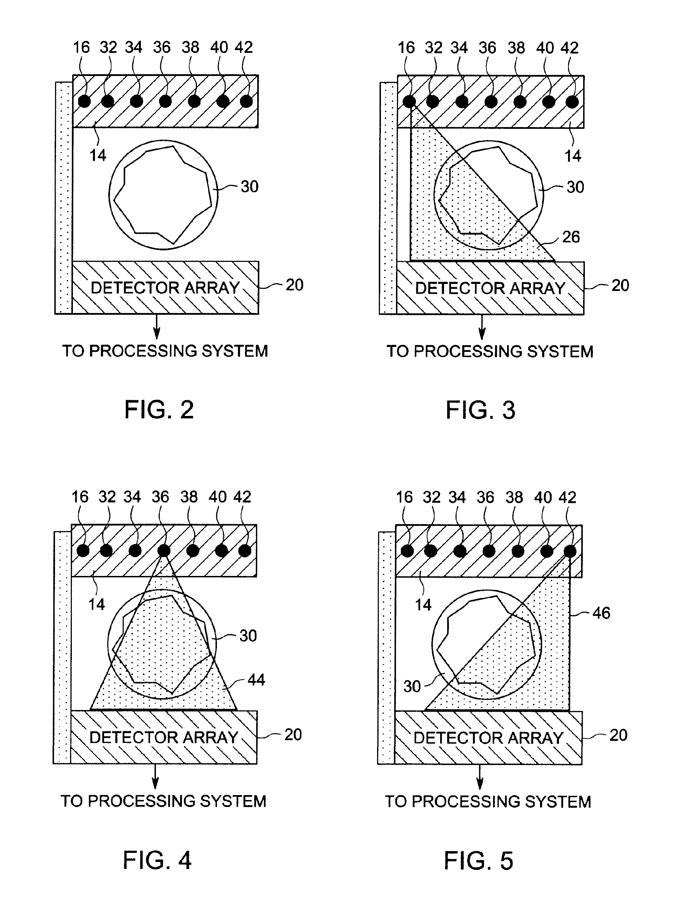

FIGS. 2-5 illustrate front views of the imaging system 10 of FIG. 1 during an example mode of operation that may be utilized to acquire a set of projection images over a given arc that may then be utilized to reconstruct the desired object slices at different planes. Specifically, FIG. 2 illustrates the initial setup prior to image acquisition. In this embodiment, the imaging setup includes the multiple focal spot x-ray source 14, the detector array 20, and a pipe 30 disposed between the x-ray source 14 and the detector 20 for imaging. The multiple focal spot x-ray source 14 includes the first focal spot 16, a second focal spot 32, a third focal spot 34, a fourth focal spot 36, a fifth focal spot 38, a sixth focal spot 40, and a seventh focal spot 42.

During operation, the x-ray source 14 is adapted to selectively activate each of the focal spots to obtain a set of projection images of the pipe 30. For example, FIG. 3 illustrates acquisition of a first projection image of the pipe 30. As shown, the first focal spot 16 is activated to produce the first x-ray beam 26. The first x-ray beam 26 is projected through the pipe 30, and the detector array 20 detects at least a portion of the x-ray beam 26. The detector 20 generates a digital representation of the detected x-rays, which is exported to the processing system for storage and processing.

FIG. 4 illustrates acquisition of a second projection image of the pipe 30, which is obtained without substantial rotation or translation of the x-ray source 14 and without substantial rotation or translation of the detector 20 or object 30. As illustrated, the fourth focal spot 36 is activated to produce a second x-ray beam 44 that is projected toward the pipe 30. The second x-ray beam 44 is attenuated by the pipe 30 before being detected by the detector array 20. As before, the detector array 20 digitizes the detected x-rays to generate a second projection image that is exported for further processing and storage.

FIG. 5 illustrates acquisition of a third projection image of the pipe 30, which is again obtained without substantial movement of the x-ray source 14, the detector 20, or the object 30. During acquisition of the third projection image, the seventh focal spot 42 is activated to produce a third x-ray beam 46 that is directed toward the pipe 30. The third x-ray beam 46 is attenuated by the pipe 30 before impinging on the detector array 20 and being converted into the third projection image. In this way, three projection images of the pipe 30 may be acquired by the imaging system without substantial movement of the multiple focal spot x-ray source 14 the detector 20, or the object 30.

In the illustrated embodiment, the three acquired projection images form a single set of images acquired over a single arc that may be utilized to reconstruct multiple planes of interest in the acquisition volume of the pipe 30. It should be noted, however, that in further embodiments, any desired number of focal spots may be provided in the stationary x-ray source 14 and activated to produce any desired number of images over the given arc range. For example, in the illustrated embodiment, each of the seven focal spots may be activated to produce seven projection images in a single acquisition set. Further, the images may be acquired over any desired angular range (e.g., 20 degrees, 30 degrees, 40 degrees, etc.). However, as described in detail above, a shift-and-add procedure may be performed on each acquisition set to reconstruct the desired planes within the pipe 30.

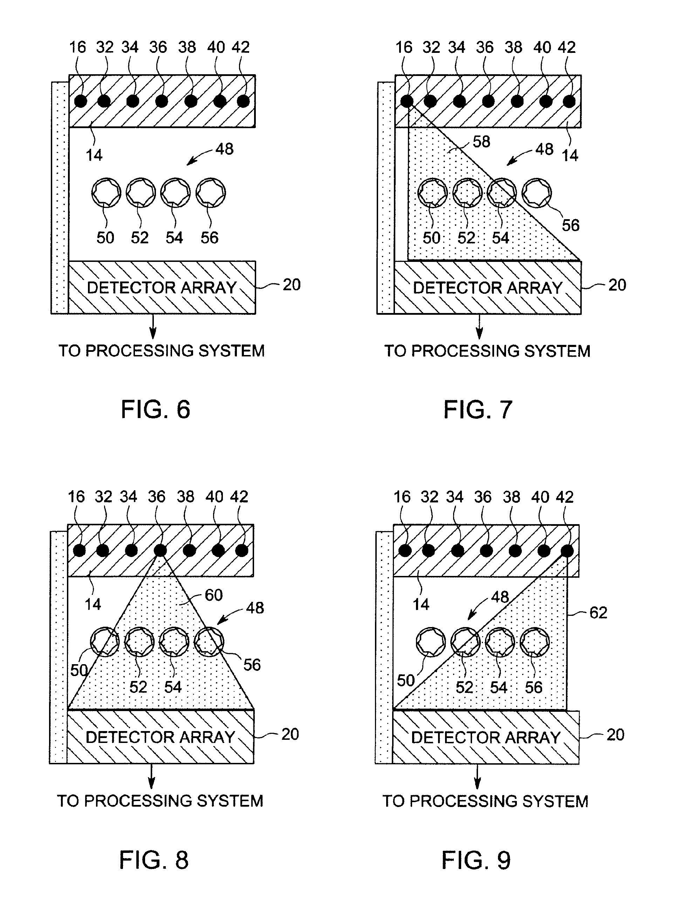

FIGS. 6-9 illustrate front views of the imaging system 10 of FIG. 1 during an example mode of operation that may be utilized to acquire a set of projection images of an array of objects. Specifically, FIG. 6 illustrates the initial setup prior to image acquisition. In this embodiment, the imaging setup includes the multiple focal spot x-ray source 14, the detector array 20, and an array of pipes 48 disposed between the x-ray source 14 and the detector 20 for imaging. The array of pipes 48 includes a first pipe 50, a second pipe 52, a third pipe 54, and a fourth pipe 56. As before, the multiple focal spot x-ray source 14 includes focal spots 16, 32, 34, 36, 38, 40, and 42.

During operation, the x-ray source 14 selectively activates each of the focal spots to obtain a set of projection images of the pipe array 48. For example, FIG. 7 illustrates acquisition of a first projection image of the pipe array 48. As shown, the first focal spot 16 is activated to produce a first x-ray beam 58. The first x-ray beam 58 is projected through the pipe array 48, and the detector array 20 detects at least a portion of the x-ray beam 58 after attenuation through pipes 50, 52, and 54. The detector 20 generates a digital representation of the detected x-rays, which is exported to the processing system for storage and subsequent processing.

FIG. 8 illustrates acquisition of a second projection image of the pipe array 48, which is obtained without substantial rotation or translation of the x-ray source 14, the detector 20, or the objects 50, 52, 54, 56, 58. As illustrated, the fourth focal spot 36 is activated to produce a second x-ray beam 60 that is projected toward the pipe array 48. The second x-ray beam 60 is attenuated by each of the pipes 50, 52, 54, and 56 in the pipe array 48 before being detected by the detector array 20. As before, the detector array 20 digitizes the detected x-rays to generate a second projection image that is exported for further processing and storage.

FIG. 9 illustrates acquisition of a third projection image of the pipe array 48, which is again obtained without substantial movement of the x-ray source 14, the detector 20, or the objects 50, 52, 54, 56, 58. During acquisition of the third projection image, the seventh focal spot 42 is activated to produce a third x-ray beam 62 that is directed toward the pipe array 48. The third x-ray beam 62 is attenuated by pipes 52, 54, and 56 before impinging on the detector array 20 and being converted into the third projection image. In this way, as before with the single pipe system of FIGS. 2-5, three projection images of the pipe array 48 may be acquired by the imaging system without substantial movement of the multiple focal spot x-ray source 14, the detector 20, or the objects 50, 52, 54, 56, 58. Additionally, in the illustrated embodiment, the three acquired projection images form a single set of images acquired over a single arc that may be utilized to reconstruct multiple planes of interest in the acquisition volume of the pipe array 48.

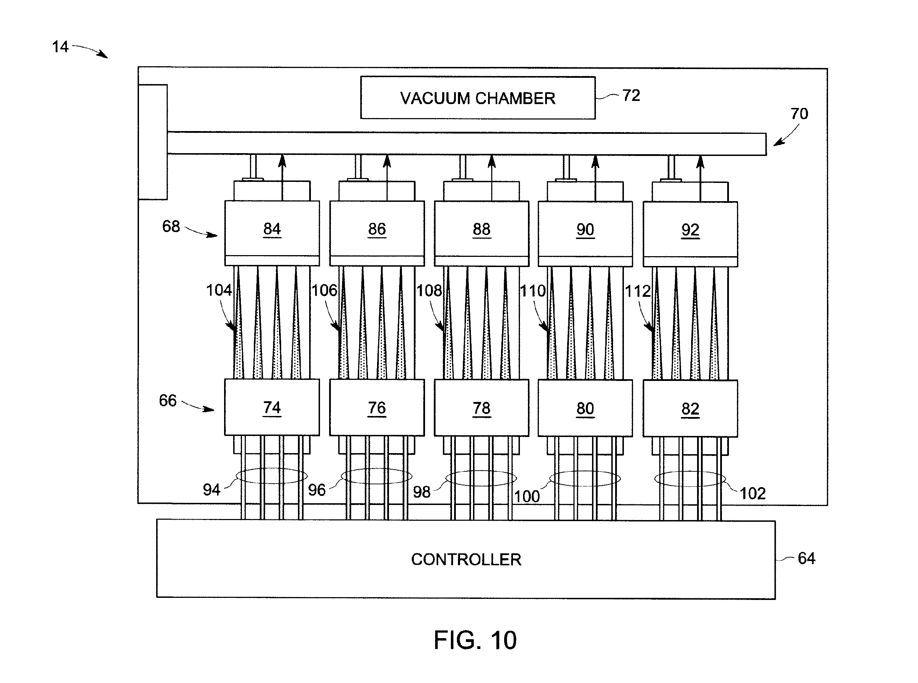

FIG. 10 illustrates an embodiment of the multiple focal spot x-ray source 14 shown in FIGS. 1-9. However, it should be noted that such an embodiment is merely an example, and any suitable multiple focal spot x-ray source may be utilized in the stationary imaging systems described herein. In the illustrated embodiment, the x-ray source 14 includes a controller 64, a plurality of cathodes 66, a plurality of anodes 68, a high voltage and oil feedthrough 70, and a vacuum chamber 72. The plurality of anodes includes a first anode 74, a second anode 76, a third anode 78, a fourth anode 80, and a fifth anode 82. The plurality of cathodes includes a first cathode 84, a second cathode 86, a third cathode 88, a fourth cathode 90, and a fifth cathode 92. The controller is coupled to the first anode 74 via control lines 94, to the second anode 76 via control lines 96, to the third anode 78 via control lines 98, to the fourth anode 80 via control lines 100, and to the fifth anode 82 via control lines 102.

During operation, the plurality of cathodes 66 emit electrons into the vacuum chamber 72, and the electrons are collected by the plurality of anodes 68, thus establishing electron beams 104, 106, 108, 110, and 112 through the x-ray tube 14. As the electrons originate from the plurality of cathodes 66 and collide with the plurality of anodes 68, energy is generated and emitted as x-rays, for example, in a direction perpendicular to the electron beams 104, 106, 108, 110, and 112. The high voltage and oil feedthrough 70 accelerates the electrons as they flow through the x-ray tube. The controller 64 controls each of the anodes individually to control x-ray generation such that the previously described sets of projection images may be acquired.

FIG. 11 illustrates a laminography inspection method 114 that may be utilized to operate the imaging system of FIG. 1. The method 114 includes providing an object to the inspection area (block 116), for example, providing a pipe to an inspection area. The method 114 also includes irradiating the object at a desired angle with a stationary multiple focal spot x-ray source (block 118) and, after the x-rays are attenuated by the object, detecting at least a portion of the x-rays on the detector array (block 120). The acquired x-ray projection data is also stored (block 122), for example, to a memory of a control or processing system. The object is then irradiated at a variety of additional angles to obtain additional projection images while maintaining the x-ray source substantially stationary (block 124).

In the illustrated method, the object is subsequently translated (block 126) and again irradiated at a plurality of angles to obtain another set of projection images at the second lengthwise location along the length of the object (block 128). However, it should be noted that during acquisition of the set of projection images, the multiple focal spot x-ray source remains substantially stationary with respect to rotational and translational movement. The acquired x-ray data along the length of the object is then processed (block 130), and a determination is made as to whether the object is accepted, rejected, or flagged (block 132). That is, the object is inspected for the presence or absence of a defect, such as stress corrosion on a pipe.

In one embodiment of the above method, if the object fits within the boundary of the source detector active regions, there may be no movement of the object necessary to obtain laminographic data and to reconstruct planes of the object. In such embodiments, the virtual motion of the source may substitute for the motion of the object. As such, certain embodiments may be substantially stationary in that no moving parts are necessary, thus possibly reducing the complexity and monetary expense associated with making and operating the disclosed imaging systems.

FIG. 12 illustrates an embodiment of the processing step of FIG. 11. The processing method 130 includes providing the acquired projection data to a processor (block 134). For example, a single set of projection data taken over a given angular range at one lengthwise location along the length of the object may be provided to the processor. The method 130 also includes shifting each acquired image within the set of projection data by a desired amount for the reconstruction of a first plane (block 136) and adding each shifted image to produce an unprocessed first plane (block 138). That is, as described above, the acquired projection images may be shifted and added to produce slices of the object at various volumetric depths. If desired, one or more deblurring or processing techniques may be utilized to remove out of focus artifacts to produce a processed first plane (block 140). The processed first plane may be compared to a reference to determine the presence or absence of a defect (block 142). To reconstruct additional planes, the projection images within a given set may again be shifted by a second desired amount, added together, and processed to produce images of additional planes at different depths through the object (block 144).

Various technical effects of embodiments of the invention can include an increased inspection speed as compared to traditional systems. That is, the multiple focal spot x-ray systems disclosed herein are capable of electronically addressing each of the focal spots in a specific manner, thus allowing each focal spot to be activated and deactivated quickly (e.g., within 1 microsecond). Such features may reduce the length of time necessary to detect and characterize a defect in an industrial product. Further, the systems disclosed herein may offer additional technical advantages over single spot x-ray sources, such as the scalability of the generated power. The total power capable of being generated by embodiments of present invention may be substantially greater than many typical single spot x-ray sources. Additionally, in embodiments in which an object size fits within the active regions of the source detector configuration, movement of the x-ray source, the object, or the detector may not be necessary, thus possibly reducing or eliminating motion blur.

This written description uses examples to disclose the invention, including the preferred mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

Various aspects and embodiments of the present invention are defined by the following numbered clauses:
1. An imaging system, comprising:
   a multiple focal spot x-ray source configured to generate at least two x-ray beams and to project each of the generated x-ray beams at a different angle one at a time toward a field of view without substantial rotation or translation of the multiple focal spot x-ray source;
   a detector opposite the multiple focal spot x-ray source relative to the field of view and configured to receive at least a fraction of the projected x-ray beams from each of the different angles and to produce at least two x-ray projection images of the field of view corresponding to each of the different angles, wherein each of the x-ray projection images are configured to be shifted with respect to one another and added to reconstruct a plane of the field of view.
2. The imaging system of clause 1, wherein the multiple focal spot x-ray source comprises an anode and a cathode and is configured to generate the at least two x-ray beams by generating a current flow between the cathode and the anode.
3. The imaging system of any preceding clause, further comprising a controller configured to inspect the reconstructed plane of the field of view to determine the presence or absence of a defect in an object within the field of view.
4. The imaging system of any preceding clause, further comprising a controller configured to activate a separate and distinct anode and cathode pair to generate each of the at least two x-ray beams.
5. The imaging system of any preceding clause, further comprising a table configured to be translated from a first position to a second position so as to move an object positioned on the table within the field of view.
6. The imaging system of any preceding clause, wherein when the table is translated from the first position to the second position, the multiple focal spot x-ray source is configured to generate and project at least two additional x-ray beams at different angles one at a time toward the object without substantial movement of the multiple focal spot x-ray source and wherein the detector detects at least a portion of the at least two additional x-ray beams after being attenuated by the object to produce an additional at least two x-ray projection images of the object.
7. The imaging system of any preceding clause, wherein an object is placed between the x-ray source and the detector such that the object is substantially covered by the field of view of the detector, and wherein the at least two projection images of the object are configured to be reconstructed to provide volumetric slices of the object.
8. A laminography inspection method, comprising:
   providing an object to an inspection area;
   irradiating the object with at least two x-ray beams, each incident on the object from a different angle, one at a time, to generate a series of angularly displaced images of the object, wherein the at least two x-ray beams are generated by a stationary multiple focal spot x-ray source;
   shifting each of the angularly displaced images with respect to one another;
   adding each of the shifted images together to reconstruct an image of a plane of the object; and
   inspecting the reconstructed image plane to identify the presence or absence of a defect in the object.
9. The method of any preceding clause, wherein the multiple focal spot x-ray source is configured to generate the at least two x-ray beams via current flow established between an anode and a cathode.
10. The method of any preceding clause, wherein inspecting the reconstructed image plane comprises comparing the reconstructed plane to a reference plane to identify one or more differences between the reconstructed plane and the reference plane.
11. The method of any preceding clause, wherein the stationary multiple focal spot x-ray source is configured to remain stationary with respect to rotational and translational movement.
12. The method of any preceding clause, wherein the series of angularly displaced images are obtained over an angle approximately equal to 40 degrees.
13. The method of any preceding clause, comprising applying a deblurring Igorithm to the reconstructed plane image to substantially reduce the background effects of out of focus planes prior to inspecting the reconstructed plane image for the presence or absence of defects.
14. The method of any preceding clause, further comprising adding each of the shifted images together to reconstruct a second image of a second plane of the object.
15. The method of any preceding clause, further comprising determining a thickness of the object based on the image of the plane of the object and the second image of the second plane of the object.
16. A laminography inspection system, comprising:
   a multiple focal spot x-ray source configured to irradiate an object with a series of angularly displaced x-ray beams one at a time without substantial rotation or translation of the multiple focal spot x-ray source, wherein the multiple focal spot x-ray source is disposed on a first side of the object;
   a detector configured to receive at least a fraction of the angularly displaced x-ray beams after being attenuated by the object to produce at least two x-ray projection images of the object, wherein the detector is disposed on a second side of the object opposite the first side; and
   a processor configured to process the at least two x-ray projection images to bring at least two planes of the object into focus, one at a time.
17. The laminography inspection system of any preceding clause, wherein the multiple focal spot x-ray source comprises a cathode and an anode and is configured to generate the angularly displaced x-ray beams by generating electrical current between the anode and the cathode.
18. The laminography inspection system of any preceding clause, wherein the detector is a digital flat panel detector.
19. The laminography inspection system of any preceding clause, wherein the processor is further configured to accept, reject, or flag the object based on a comparison of the at least two focused planes with a reference.
20. The laminography inspection system of any preceding clause, wherein the object is at least one of a pipe, a pipe array, a wind blade, a wind blade spar cap, and a printed circuit board with discrete components.
21. The laminography inspection system of any preceding clause, wherein the processor is configured to shift and add the at least two projection images to bring the at least two planes of the object into focus, one at a time.

## Claims

1. An imaging system (10), comprising:
a multiple focal spot x-ray source (14) configured to generate at least two x-ray beams (26) and to project each of the generated x-ray beams (26) at a different angle one at a time toward a field of view without substantial rotation or translation of the multiple focal spot x-ray source (14);
a detector (20) opposite the multiple focal spot x-ray source (14) relative to the field of view and configured to receive at least a fraction of the projected x-ray beams (26) from each of the different angles and to produce at least two x-ray projection images of the field of view corresponding to each of the different angles, wherein each of the x-ray projection images are configured to be shifted with respect to one another and added to reconstruct a plane of the field of view.

2. The imaging system (10) of claim 1, wherein the multiple focal spot x-ray source (14) comprises an anode (68) and a cathode (66) and is configured to generate the at least two x-ray beams (104, 106, 108, 110, and 112) by generating a current flow between the cathode (66) and the anode (68).

3. The imaging system (10) of any preceding claim, further comprising a controller (22) configured to inspect the reconstructed plane of the field of view to determine the presence or absence of a defect in an object (12) within the field of view.

4. The imaging system (10) of any preceding claim, further comprising a controller (64) configured to activate a separate and distinct anode (84) and cathode (74) pair to generate each of the at least two x-ray beams (104 and 106).

5. The imaging system (10) of any preceding claim, further comprising a table (18) configured to be translated from a first position to a second position so as to move an object (12) positioned on the table (18) within the field of view.

6. The imaging system (10) of claim 5, wherein when the table (18) is translated from the first position to the second position, the multiple focal spot x-ray source (14) is configured to generate and project at least two additional x-ray beams at different angles one at a time toward the object (12) without substantial movement of the multiple focal spot x-ray source (14) and wherein the detector (20) detects at least a portion of the at least two additional x-ray beams after being attenuated by the object (12) to produce an additional at least two x-ray projection images of the object (12).

7. The imaging system (10) of any preceding claim, wherein an object (12) is placed between the x-ray source (14) and the detector (20) such that the object (12) is substantially covered by the field of view of the detector (20), and wherein the at least two projection images of the object (12) are configured to be reconstructed to provide volumetric slices of the object (12).

8. A laminography inspection method (114), comprising:
providing (116) an object (12) to an inspection area (18);
irradiating (118) the object (12) with at least two x-ray beams (26), each incident on the object from a different angle, one at a time, to generate a series of angularly displaced images of the object, wherein the at least two x-ray beams (26) are generated by a stationary multiple focal spot x-ray source (14);
shifting (136) each of the angularly displaced images with respect to one another;
adding (138) each of the shifted images together to reconstruct an image of a plane of the object (12); and
inspecting (132) the reconstructed image plane to identify the presence or absence of a defect in the object (12).

9. The method (114) of claim 8, wherein the multiple focal spot x-ray source (12) is configured to generate the at least two x-ray beams (26) via current flow established between an anode (68) and a cathode (66).

10. The method (114) of claim 8 or claim 9, wherein inspecting (132) the reconstructed image plane comprises comparing (142) the reconstructed plane to a reference plane to identify one or more differences between the reconstructed plane and the reference plane.

11. The method (114) of any of claims 8 to 10, wherein the stationary multiple focal spot x-ray source (14) is configured to remain stationary with respect to rotational and translational movement.

12. The method (114) of any of claims 8 to 11, wherein the series of angularly displaced images are obtained over an angle approximately equal to 40 degrees.

13. The method (114) of any of claims 8 to 12, comprising applying (140) a deblurring algorithm to the reconstructed plane image to substantially reduce the background effects of out of focus planes prior to inspecting (132) the reconstructed plane image for the presence or absence of defects.

14. The method (114) of any of claims 8 to 13, further comprising adding (138) each of the shifted images together to reconstruct a second image of a second plane of the object (12).

15. The method (114) of any of claims 8 to 14, further comprising determining (130) a thickness of the object (12) based on the image of the plane of the object and the second image of the second plane of the object (12).
